Europäisches Patentamt

(19) European Patent Office

Office européen des brevets

(11) Numéro de publication : **0 229 134**
**B1**

(12) FASCICULE DE BREVET EUROPÉEN

(45) Date de publication du fascicule du brevet :
11.10.89

(21) Numéro de dépôt : 86904212.7

(22) Date de dépôt : 08.07.86

(86) Numéro de dépôt international :
PCT/FR 86/00244

(87) Numéro de publication internationale :
WO/8700487 (29.01.87 Gazette 87/03)

(51) Int. Cl.⁴ : **B 43 M 11/08**

(54) DISPOSITIF POUR L'APPLICATION D'UNE QUANTITE PREDETERMINEE D'UN LIQUIDE SUR UNE FEUILLE.

(30) Priorité : 17.07.85 FR 8510939

(43) Date de publication de la demande :
22.07.87 Bulletin 87/30

(45) Mention de la délivrance du brevet :
11.10.89 Bulletin 89/41

(84) Etats contractants désignés :
AT DE GB IT

(56) Documents cités :
EP—A— 0 099 706
CH—A— 256 553
GB—A— 643 626
GB—A— 2 001 285
US—A— 4 080 078
US—A— 4 389 132

(73) Titulaire : **Kores Holding Zug AG**
**Baarer Strasse 57**
**CH-6300 Zug (CH)**

(72) Inventeur : **KORESKA, Robert**
**24, rue Marc Seguin**
**F-75018 Paris (FR)**
Inventeur : **DE SAINT-LEON, André**
**71, avenue de la Victoire**
**F-77109 Meaux (FR)**

(74) Mandataire : **Büchel, Kurt F., Dr.**
**Patentanwalt Dr. Kurt F. Büchel Bergstrasse 297**
**FL-9495 Triesen (LI)**

Jouve, 18, rue St-Denis, 75001 Paris, France

## Description

La présente invention concerne un dispositif selon le préambule de la revendication 1.

Lorsque l'on désire appliquer manuellement une quantité relativement limitée d'un liquide sur une surface, par exemple quelques millilitres d'un solvant, d'une colle liquide ou d'un vernis, la méthode la plus simple consiste à utiliser un applicateur, du type pinceau, que l'on trempe dans le liquide à appliquer, et que l'on passe ensuite sur la surface à enduire ou à nettoyer pour y déposer un film de liquide.

Cette méthode implique l'utilisation d'un récipient contenant une quantité relativement importante de liquide et entraîne des pertes considérables de liquide ainsi qu'une application irrégulière.

Pour permettre l'application d'une quantité prédéterminée de liquide on a proposé d'utiliser des doses unitaires, par exemple sous forme d'une ampoule ou d'une capsule frangible, contenant une quantité déterminée de liquide, que l'on brise pour répandre le liquide sur un matériau absorbant ou sur un pinceau, permettant son étalement sur la surface. Toutefois la rupture de l'ampoule ou de la capsule contenant le liquide est souvent malaisée, elle ne se produit pas toujours à l'endroit voulu, et elle entraîne des risques de blessure pour l'utilisateur par contact avec des débris de l'ampoule ou avec le liquide libéré.

Le brevet anglais 2.001.285 décrit un dispositif pour l'application d'une colle liquide, comprenant un piston permettant de percer et comprimer une cartouche pour faire sortir la colle par une ouverture masquée par une bille. Dans le brevet US 4.389.132, le liquide à appliquer est également contenu dans une cartouche en matière plastique percée par un tube. Ces dispositifs connus sont cependant relativement complexes et n'évitent pas un encrassement de l'élément d'application du liquide sur la surface à enduire.

La présente invention a pour objet un dispositif simple, de fabrication peu coûteuse, permettant d'appliquer par enduction sur une surface, une quantité prédéterminée d'un liquide contenu dans une ampoule ou une capsule frangible, facilement et dans de bonnes conditions de sécurité.

Le dispositif conforme à la présente invention est du type comportant une dose de liquide dans une ampoule ou capsule frangible, et se caractérise par les caractéristiques de la revendication 1.

Les languettes flexibles peuvent être avantageusement réalisées par simple découpe dans le corps tubulaire.

La capsule ou ampoule frangible a de préférence une forme cylindrique allongée de manière à pouvoir être introduite dans le corps tubulaire par son extrémité ouverte. Elle est enfermée dans une gaine en matière souple et résistante, de même forme sensiblement cylindrique fermée à une extrémité par un matériau absorbant faisant fonction de mèche ou de tampon applicateur, qui dépasse hors du corps tubulaire lorsque l'ampoule ou la capsule y est mise en place.

A son extrémité opposée à l'ouverture, le corps tubulaire comporte de préférence un poussoir, constitué par exemple par un piston, pouvant coulisser librement suivant l'axe longitudinal. Ce poussoir est repoussé lors de la mise en place de la capsule ou ampoule dans le corps tubulaire, de telle sorte qu'une tige solidaire du poussoir dépasse hors du corps tubulaire, à son extrémité opposée à l'ouverture. Cette tige peut être actionnée ensuite pour repousser le poussoir et éjecter la capsule ou ampoule hors du corps tubulaire, après utilisation du dispositif de l'invention.

Lorsque la capsule ou ampoule est mise en place dans le corps tubulaire, elle repousse vers l'extérieur les ergots et les languettes flexibles sur lesquelles ils sont fixés. En exerçant une pression sur les languettes, les ergots en forme de pointe, décalés longitudinalement l'un par rapport à l'autre, exercent un effet de cisaillement qui provoque la cassure de l'ampoule à travers la gaine, et empêchent la gaine de glisser hors du corps tubulaire. Le liquide contenu dans l'ampoule est ainsi mis directement en contact avec le matériau absorbant qui dépasse hors du corps tubulaire.

Le dispositif de l'invention, constitué du corps tubulaire et de la gaine renfermant l'ampoule brisée, peut alors être utilisé comme un pinceau ou un tampon applicateur pour déposer un film de liquide sur la surface à traiter. Lors de cette opération de cassure de l'ampoule et d'application du liquide, l'utilisateur tient le dispositif par le corps tubulaire, ce qui évite tout risque de blessure ou de salissure par un débris de l'ampoule ou par le liquide qu'elle contient. De plus, en fin d'utilisation, il suffit d'agir sur la tige du poussoir pour éjecter l'ampoule et sa gaine sans contact avec les doigts.

L'ampoule est de préférence en verre résistant au liquide qu'elle contient. La matière constituant la gaine peut être toute matière souple présentant une résistance suffisante pour ne pas se déchirer sous l'action des ergots. On peut utiliser par exemple du polyéthylène.

Le corps tubulaire peut être réalisé en métal léger, en alliage ou en matière plastique telle qu'un polypropylène, du nylon, un polystyrène, etc. Il peut être avantageux d'utiliser un nylon renforcé de fibres de verre pour le corps tubulaire, et un matériau plus dur, par exemple du polypropylène, pour le poussoir.

Le dispositif suivant l'invention peut être utilisé pour appliquer un liquide sur une surface, notamment pour nettoyer des caractères de machines à écrire au moyen d'un solvant tel qu'un alcool ou du trichloroéthylène. On peut également l'utiliser pour appliquer un vernis protecteur sur une surface. Bien entendu, le tampon applicateur peut être modifié librement en fonction du liquide utilisé et des conditions d'utilisation du dispositif.

Les caractéristiques et avantages de l'invention apparaîtront plus en détail dans la description ci-après relative à un exemple non limitatif de réalisation, en référence aux dessins annexés qui représentent :

Figure 1 : une coupe longitudinale d'un dispositif de sectionnement conforme à la présente invention.

Figure 2 : une coupe longitudinale du dispositif de la figure 1, renfermant une ampoule de liquide dans sa gaine, prêt à être utilisé.

Figure 3 : une vue de dessus du dispositif de la figure 1.

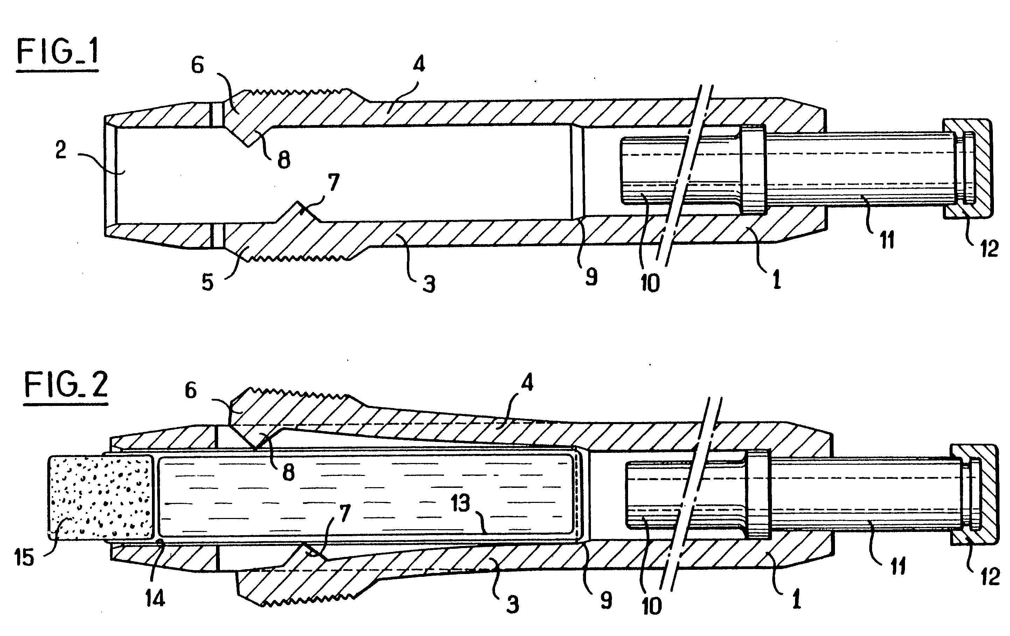

Comme le montre la figure 1, le dispositif comprend un corps tubulaire (1) sensiblement cylindrique, ouvert à une extrémité (2). Dans ce corps cylindrique (1) sont découpées deux languettes (3) et (4) flexibles dont les extrémités, (5) et (6) respectivement, sont libres et portent chacune un ergot (7) et (8) respectivement, en forme de pointe, moulé dans la matière de la languette.

L'intérieur du corps tubulaire (1) présente un épaulement annulaire (9) servant à centrer et maintenir la gaine renfermant l'ampoule que l'on introduit par l'ouverture (2).

A son extrémité opposée à l'ampoule (2), le corps tubulaire (1) comporte un poussoir (10) en forme de piston coulissant librement suivant l'axe longitudinal du corps tubulaire, et dont la tige (11) traverse le corps (1) à son extrémité. La tige (11) est coiffée d'un capuchon (12) facilitant son actionnement et empêchant sa sortie du corps (1).

Lorsque l'ampoule est mise en place dans le corps tubulaire (1) par son ouverture (2), comme le montre la figure 2, elle repousse les ergots (7) et (8) et les languettes (3) et (4) qui les portent, son centrage est assuré par l'épaulement (9) dont la position est prévue en fonction de la dimension de l'ampoule et de sa gaine et en position extrême, elle peut venir en butée contre le poussoir (10). Selon sa forme et sa dimension, l'ampoule est en contact avec le poussoir (10) ; dans des conditions usuelles d'utilisation, il suffit toutefois qu'elle soit maintenue par l'épaulement (9). L'ampoule (13) en verre est enfermée dans une gaine (14) flexible et résistante dont l'extrémité dépassant par l'ouverture (2) est fermée par un tampon applicateur (15) absorbant.

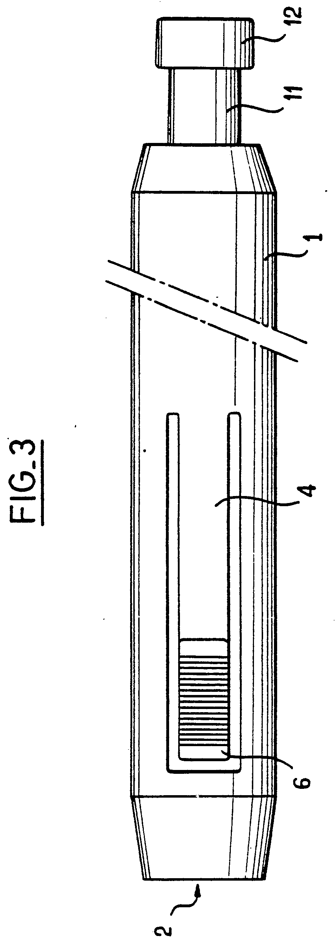

Une pression sur les deux languettes (3) et (4) formées par découpe dans le corps tubulaire (1) comme représenté sur la figure 3, provoque un effet de cisaillement sur l'ampoule (13) à travers sa gaine (14) en raison de la position décalée longitudinalement des ergots (7) et (8) l'un par rapport à l'autre. Le liquide contenu dans l'ampoule (13) se répand alors à l'intérieur de la gaine flexible (14) et vient au contact du tampon absorbant (15) qui se trouve alors imbibé. Après usage, en agissant sur le capuchon (12) on repousse la tige (11) et le poussoir (10) et on éjecte l'ampoule (13) brisée, sa gaine (14) et le tampon (15).

## Revendications

1. Dispositif pour l'application, par enduction sur une surface, d'une quantité prédéterminée d'un liquide contenu dans une ampoule (13) ou une capsule frangible, ce dispositif comprenant un corps tubulaire (1) ouvert à une extrémité (2) recevant l'ampoule (13) ou capsule frangible sensiblement cylindrique d'un moyen d'application du liquide et d'un moyen (3, 4) pour rompre l'ampoule (13) ou capsule frangible, caractérisé en ce que l' ampoule (13) ou capsule frangible est contenue dans une gaine (14) flexible résistante, et que le corps tubulaire recevant l'ampoule ou capsule comporte dans sa paroi au moins deux languettes flexibles (3, 4) disposée sensiblement en face l'une de l'autre, chacune de ces languettes présentant au moins un ergot (7, 8) orienté vers l'intérieur, et décalé longitudinalement par rapport à l'ergot correspondant de l'autre languette, de sorte qu'une pression sur les deux languettes provoque un effet de cisaillement sur l'ampoule ou capsule à travers la gaine et que le liquide contenu dans l'ampoule se répande à l'intérieur de la gaine flexible vers le moyen d'application (15).

2. Dispositif selon la revendication 1, caractérisé en ce que les languettes flexibles sont réalisées par simple découpe dans le corps tubulaire.

3. Dispositif selon la revendication 1, caractérisé en ce que l'ampoule (13) ou capsule frangible est contenue dans une gaine (14) flexible résistante fermée à une extrémité par un matériau absorbant (15) qui dépasse hors du corps tubulaire (1) lorsque l'ampoule (13) ou la capsule y est mise en place.

4. Dispositif selon la revendication 1, caractérisé en ce qu'il comporte, à son extrémité opposée à l'ouverture, un poussoir (10) solidaire d'une tige (11) dépassant hors du corps tubulaire (1).

5. Dispositif selon la revendication 1, caractérisé en ce que le corps tubulaire (1) présente sur sa paroi intérieure un épaulement annulaire (9) permettant le centrage et le maintien de la gaine (14).

## Claims

1. A device for applying by coating to a surface a predetermined amount of a liquid contained in a frangible ampoule (13) or capsule, said device comprising a tubular body (1) open at one end (2) for receiving the substantially cylindrical frangible ampoule (13) or capsule, a means for applying the liquid and a means (3, 4) for breaking the frangible ampoule (13) or capsule, characterised in that the frangible ampoule (13) or capsule is contained in a strong flexible sheath (14) and that the tubular body receiving the ampoule or capsule comprises in its wall at least two flexible tongues (3, 4) which are disposed substantially facing each other, each of said tongues having at least one inwardly directed lug (7, 8) which is longitudinally displaced with respect to the corresponding lug of the other tongue, in such a way that a pressure on the two tongues produces a shearing action on the ampoule or capsule through the sheath and the liquid contained in the ampoule spreads out within the flexible sheath towards the application means (15).

2. A device according to claim 1 characterised in that the flexible tongues are produced by being simply cut out in the tubular body.

3. A device according to claim 1 characterised in that the frangible ampoule (13) or capsule is contained in a strong flexible sheath (14) closed at one end by an absorbent material (15) which projects out of the tubular body (1) when the ampoule (13) or the capsule is set in place therein.

4. A device according to claim 1 characterised in that at its end opposite to the opening it comprises a thrust member (10) fixed with respect to a rod (11) which projects out of the tubular body (1).

5. A device according to claim 1 characterised in that on its inward wall surface the tubular body (1) has an annular shoulder (9) for centering and holding the sheath (14).

**Patentansprüche**

1. Vorrichtung zum Anbringen einer bestimmten Menge einer in einer Ampulle oder zerbrechlichen Kapsel enthaltenen Flüssigkeit auf eine Oberfläche in Form einer dünnen Schicht, wobei diese Vorrichtung einen röhrenförmigen Hohlkörper (1) mit einer Öffnung (2) zur Aufnahme der zylinderförmigen Ampulle (13) bzw. der zerbrechlichen Kapsel, eine Auftrageeinrichtung für die Flüssigkeit und Vorrichtungen zum Zerbrechen der Ampulle (13) bzw. der zerbrechlichen Kapsel aufweist, dadurch gekennzeichnet, dass die Ampulle (13) bzw. die zerbrechliche Kapsel in einer elastischen, widerstandsfähigen Hülle (14) enthalten ist und dass der die Ampulle (13) bzw. die zerbrechliche Kapsel aufnehmende röhrenförmige Hohlkörper (1) an seinen Wänden wenigstens zwei elastische, einander gegenüberliegende Laschen (3 ; 4) aufweist, wobei jede dieser Laschen (3 ; 4) wenigstens einen gegen das Innere gerichteten Vorsprung (7 ; 8) aufweist, der gegen den entsprechenden Vorsprung der anderen Lasche längsversetzt angeordnet ist, so dass beim Druck auf die beiden Laschen (3 ; 4) ein scherendes Zerschneiden der Ampulle (13) bzw. der Kapsel durch die Hülle (14) hindurch stattfindet und die in der Ampulle (13) enthaltene Flüssigkeit sich im Inneren der elastischen Hülle (14) gegen die Appliziervorrichtung (15) zu ausbreitet.

2. Vorrichtung nach Anspruch 1, dadurch gekennzeichnet, dass die elastischen Laschen (3 ; 4) aus dem röhrenförmigen Hohlkörper (1) ausgeschnitten sind.

3. Vorrichtung nach Anspruch 1, dadurch gekennzeichnet, dass die Ampulle (13) bzw. die zerbrechliche Kapsel in einer elastischen, widerstandsfähigen Hülle (14) enthalten ist, die an ihrem einen Ende durch ein absorbierendes Material abgeschlossen wird, das aus dem röhrenförmigen Hohlkörper (1) herausragt, wenn die Ampulle (13) bzw. die Kapsel dort eingesetzt ist.

4. Vorrichtung nach Anspruch 1, dadurch gekennzeichnet, dass der röhrenförmige Hohlkörper (1) an seinem der Öffnung (2) entgegengesetzten Ende einen Stempel (10) aufweist, der mit einem Schaft (11) fest verbunden ist, der aus dem röhrenförmigen Hohlkörper (1) herausragt.

5. Vorrichtung nach Anspruch 1, dadurch gekennzeichnet, dass der röhrenförmige Hohlkörper (1) an seiner Innenwand einen ringförmigen Absatz (9) zum zentrierenden Halt für die Hülle (14) aufweist.

FIG_1

FIG_2

FIG_3

EP 0 229 134 B1